(19) Europäisches Patentamt
European Patent Office
Office européen des brevets

(11) Veröffentlichungsnummer : **0 252 408 B1**

# (12) EUROPÄISCHE PATENTSCHRIFT

(45) Veröffentlichungstag der Patentschrift : **09.01.91 Patentblatt 91/02**

(51) Int. Cl.$^5$ : **C07C 51/58**, C07C 53/42, C07C 53/44

(21) Anmeldenummer : **87109364.7**

(22) Anmeldetag : **30.06.87**

## (54) Verfahren zur Herstellung von Carbonsäurehalogeniden.

(30) Priorität : **11.07.86 DE 3623422**

(43) Veröffentlichungstag der Anmeldung : **13.01.88 Patentblatt 88/02**

(45) Bekanntmachung des Hinweises auf die Patenterteilung : **09.01.91 Patentblatt 91/02**

(84) Benannte Vertragsstaaten : **CH DE ES FR GB IT LI NL**

(56) Entgegenhaltungen :
**EP-A- 0 070 463**
**US-A- 2 411 982**
**US-A- 3 471 557**

(73) Patentinhaber : **BASF Aktiengesellschaft**
**Carl-Bosch-Strasse 38**
**D-6700 Ludwigshafen (DE)**

(72) Erfinder : **Bott, Kaspar, Dr.**
**Werderstrasse 57**
**D-6800 Mannheim 1 (DE)**

EP 0 252 408 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Carbonsäurehalogeniden durch Umsetzung von sekundären oder tertiären Alkylhalogeniden mit Kohlenmonoxid bei erhöhtem Druck in Gegenwart katalytischer Mengen einer Lewis-Säure und gegebenenfalls in Gegenwart eines Lösungsmittels.

Aus der DE-OS 3 128 445 geht hervor, daß sich sekundäre oder tertiäre Alkylhalogenide mit Kohlenmonoxid in Gegenwart von Aluminiumchlorid oder Eisenchlorid als Katalysator zu den entsprechenden Säurehalogeniden umsetzen lassen, ohne daß äquimolere Mengen des Katalysators benötigt werden. Gute Ausbeuten und Selektivitäten werden insbesondere in Gegenwart von Aluminiumchlorid erhalten, so daß die Verwendung dieses Katalysators, jedenfalls wenn keine weiteren Brönsted- oder Lewis-Säuren zugesetzt werden, für das gute Gelingen dieses Verfahrens essentiell erscheint. Die Verwendung von Aluminiumchlorid ist insofern nachteilig, als dessen Löslichkeit in vielen für Friedel-Crafts-Synthesen bevorzugten Lösungsmitteln wie Methylenchlorid, Chloroform, Tetrachlorethylen oder Trichlorbenzol nur gering ist. Lösungsmittel, in denen Aluminiumchlorid gut löslich ist, wie Nitrobenzol oder Sulfolan, wirken hingegen desaktivierend auf den Katalysator.

Für eine in der Technik bevorzugte kontinuierliche Reaktionsführung ist es wichtig, daß ein Katalysator nicht in fester Form, sondern gelöst in den Druckreaktor eingespeist werden kann, wobei seine Aktivität natürlich nicht herabgesetzt werden sollte. Der Erfindung lag daher die Aufgabe zugrunde, einen Katalysator zu finden, bei dem die geschilderten Nachteile nicht auftreten.

Demgemäß wurde ein Verfahren zur Herstellung von Carbonsäurehalogeniden der Formel I

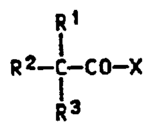 I.

in der $R^1$ Wasserstoff oder einen gegebenenfalls halogenierten Alkyl- oder Cycloalkylrest bedeutet, $R^2$ und $R^3$ einen gegebenenfalls halogenierten Alkyl- oder Cycloalkylrest darstellen, wobei $R^2$ und $R^3$ auch miteinander zu einem 5- bis 7-gliedrigen Ring verbunden sein können und X für Halogen steht, durch Umsetzung von Alkylhalogeniden der Formel II

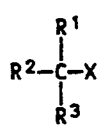 II

mit Kohlenmonoxid bei erhöhtem Druck in Gegenwart katalytischer Mengen einer Lewis-Säure gefunden, das dadurch gekennzeichnet ist, daß man die Umsetzung in Gegenwart von

a) Aluminiumbromid als Katalysator oder

b) Aluminiumbromid oder -chlorid als Katalysator sowie in Gegenwart von Halogenkohlenwasserstoffen und Carbonsäurehalogeniden der Formel III

$$R^4\text{–}CH_2\text{–}CO\text{–}Hal \quad III$$

in der $R^4$ Wasserstoff oder einen $C_1$-$C_4$-Alkylrest bedeutet und Hal für Halogen steht,
vornimmt und gegebenenfalls den Katalysator anschließend durch Zugabe eines Carbonsäureamids zerstört.

Das Aluminiumbromid, das vorteilhaft in Konzentrationen von 0,005 bis 0,05 mol pro Mol Alkylhalogenid II eingesetzt wird, ist in den für die Umsetzung besonders geeigneten Lösungsmitteln wie halogenierten Kohlenwasserstoffen gut löslich. Halogenierte Kohlenwasserstoffe sind beispielsweise bromierte, vorzugsweise chlorierte Kohlenwasserstoffe, insbesondere chlorierte Alkane, Alkene, Cycloalkane oder Benzol. Hier seien z.B. Methylenchlorid, Chloroform, Tri- und Tetrachlorethan, Tetrachlorethylen, Hexachlorbutadien sowie Mono-, Di- und Trichlorbenzol genannt. Besonders bevorzugt werden chlorierte Alkane bzw. Alkene, insbesondere solche mit 1 bis 4 Kohlenstoffatomen, vorzugsweise Methylenchlorid und Tetrachlorethylen verwendet. Die Umsetzung kann aber auch ohne Lösungsmittel durchgeführt werden, wenn das

Alkylhalogenid II bei der gewählten Reaktionstemperatur flüssig ist.

Die ohnehin gute Löslichkeit des Aluminiumbromids in den genannten Lösungsmitteln läßt sich noch weiter steigern, wenn man Carbonsäurehalogenide der Formel $R^4$-$CH_2$-CO-Hal, worin $R^4$ Wasserstoff oder einen $C_1$-$C_4$-Alkylrest und Hal Halogen, insbesondere Chlor oder Brom bedeuten, als lösungsvermittelnde Komponente zusetzt. Besonders geeignet sind $C_2$-und $C_3$-Säurehalogenide, z.B. Acetyl- und Propionylchlorid. Durch den Zusatz dieser Lösungsvermittler ist es möglich, auch Aluminiumchlorid als Katalysator zu verwenden, ohne daß Löslichkeitsprobleme auftreten.

Die Menge der Säurehalogenide kann etwa 0,1 bis 2 mol pro Mol Aluminiumhalogenid betragen. Vorzugsweise können pro Mol Aluminiumbromid 0,1 bis 1,5, insbesondere 0,3 bis 1,3 mol Säurehalogenid und pro Mol Aluminiumchlorid 0,5 bis 1,8, insbesondere 0,8 bis 1,5 mol Säurehalogenid verwendet werden.

Durch den Säurehalogenidzusatz kommt es zu keiner signifikanten Abnahme der Katalysatoraktivität und es tritt keine Decarbonylierung auf, wie es z.B. der Fall sein kann, wenn man die bei der Umsetzung gebildeten Produkte als Lösungsvermittler verwendet.

Als Ausgangsstoff II kommen Alkylhalogenide in Betracht, in denen das Halogenatom X beispielsweise ein Fluor-, Chlor- oder Bromatom darstellt. Vorteilhaft werden Alkylbromide. besonders vorteilhaft Alkylchloride umgesetzt. Der Rest $R^1$ steht für Wasserstoff sowie verzweigte oder bevorzugt unverzweigte Alkylreste, die gegebenenfalls durch ein oder mehrere Halogenatome, wie Fluor-, insbesondere Chlor- oder Bromatome, substituiert sind. Die Kohlenstoffzahl der Alkylreste kann beispielsweise 1 bis 20, vorzugsweise 1 bis 10, insbesondere 1 bis 5, betragen. Folgende Reste $R^1$ seien beispielhaft genannt : die Methyl-, Ethyl-, Propyl-, Isopropyl-, Butyl-, Isobutyl-, Amyl-, Isoamyl-, Hexyl-, Octyl- oder Dodecylgruppe sowie als halogensubstituierte Alkylreste die Chlormethyl-, Brommethyl-, Fluormethyl- oder eine durch ein Fluor-, Chlor- oder Bromatom substituierte Ethyl-, Propyl-, Butyl-, Amyl-, Hexyl-, Heptyl-, Octyl-, Decyl-oder Dodecylgruppe.

Weiterhin kann $R^1$ eine gegebenenfalls durch ein oder mehrere der oben genannten Halogenatome substituierte Cycloalkylgruppe, vorzugsweise mit 5 oder 6 Kohlenstoffatomen, bedeuten. Beispielhaft seien die Cyclobutyl-, Cyclopentyl-, Cyclohexyl-, Methylcyclohexyl-, Chlorcyclopentyl-, Chlorcyclohexyl-, Bromcyclopentyl- oder Bromcyclohexylgruppe genannt.

Die Reste $R^2$ und $R^3$ haben, abgesehen von Wasserstoff, die für $R^1$ genannte Bedeutung. Weiterhin können sie auch miteinander zusammen mit dem C-Atom, an das sie gebunden sind, ein Ringsystem bilden, das auch überbrückt sein kann. Im allgemeinen sind sie miteinander zu einem 5- bis 7-gliedrigen Ring verknüpft. Beispielsweise seien das Cyclopentyl-, Cyclohexyl-, Cycloheptyl-, Bicyclo[2,2,1]heptyl-, Bicyclo[2,2,2]octyl- oder Bicyclo[3,2,1]octylsystem genannt. Die genannten Cycloalkylreste können noch durch $C_1$-bis $C_4$-Alkylreste substituiert sein.

Für die Umsetzung können beispielsweise die folgenden Ausgangsstoffe verwendet werden : tert.-Butylchlorid und -bromid, tert.-Amylchlorid und -bromid, 1,2-Dichlor-2-methylpropan, 2-Chlor-2-methylhexan, 2-Brom-2-methylhexan, 2-Chlor-2-propylhexan, 1-Chlor-1-methylcyclohexan, Norbornylchlorid oder Norbornylbromid.

Die Umsetzung erfolgt in an sich bekannter Weise diskontinuierlich oder bevorzugt kontinuierlich bei einem Kohlenmonoxiddruck von etwa 10 bis 500, vorzugsweise 50 bis 400, insbesondere 150 bis 300 bar und einer Temperatur von –20 bis +80, vorzugsweise 0 bis 40, insbesondere 0 bis 10°C, wobei die optimale Reaktionstemperatur stark vom eingesetzten Ausgangsstoff II und Lösungsmittel abhängt. Die Menge des Lösungsmittels kann z.B. 10 bis 500 insbesondere 20 bis 100, vorteilhaft 20 bis 50 Vol.%, bezogen auf das Volumen des eingesetzten Alkylhalogenids betragen.

Nach beendeter Umsetzung kann wie bei herkömmlichen Verfahren auf Normaldruck entspannt werden, um das gebildete Produkt 1, nicht umgesetzten Ausgangsstoff II und gegebenenfalls vorhandenes Lösungsmittel vom Katalysator abzutrennen. Dabei besteht aber die Gefahr, daß sich ein Teil des Carbonsäurehalogenids in Umkehrung seiner Bildungsreaktion zersetzt. Diese Zersetzung läßt sich weitgehend vermeiden, wenn man dem Reaktionsgemisch vor dem Entspannen ein Carbonsäureamid zusetzt, um den Lewis-Säure-Katalysator zu zerstören. Vorzugsweise wird man Säureamide von niedermolekularen Carbonsäuren verwenden, die sich leicht aus dem Reaktionsgemisch abtrennen lassen, z.B. von $C_1$- bis $C_5$-Carbonsäuren, wie Ameisensäure, Essigsäure, Propionsäure, Buttersäure oder Valeriansäure. Besonders bevorzugtes Säureamid ist Dimethylformamid. Zur völligen Zerstörung des Katalysators wird man dem Aluminiumhalogenid äquimolare Mengen des Säureamids zusetzen. Kleinere und größere Mengen sind möglich, bringen aber keine Vorteile.

Die destillative Aufarbeitung des Reaktionsgemisches erfolgt in bekannter Weise, so daß sich Ausführungen hierzu erübrigen.

Beispiele 1 bis 3

Das Aluminiumbromid wurde ggf. zusammen mit dem Lösungsmittel bzw. Lösungsvermittler im Rührautoklaven bei +5°C vorgelegt. Dann wurde Kohlenmonoxid bis zu einem Druck von 200 bar aufgepreßt, das Alkylchlorid innerhalb von 30 bis 80 Minuten zugepumpt und der CO-Druck auf 300 bar erhöht. Anschließend ließ man die Reaktionsmischung 10 bis 12 h bei der angegebenen Temperatur ausreagieren, wobei der CO-Druck konstant bei 300/bars gehalten wurde.

Die Aufarbeitung erfolgte in der Weise, daß man dem Reaktionsgemisch vor dem Entspannen die dem Aluminiumbromid äquimolare Menge Dimethylformamid zusetzte und den Reaktoraustrag im Vakuum vom Katalysator abdestillierte Die durch gaschromatographische Analyse des Destillats ermittelten Ausbeuten sind ebenso wie Einzelheiten zur Umsetzung der nachfolgenden Tabelle zu entnehmen.

Tabelle

$$RCl + CO \xrightarrow{AlBr_3} R\text{-}CO\text{-}Cl$$

| Beispiel | RCl g/mol | $AlBr_3$ g/mol | Lösungsmittel g | Abreaktion bei T = °C | Ausbeute[a)] g/% | Selektivität[a)] % |
|---|---|---|---|---|---|---|
| 1 | exo-Norbornylchlorid 132/1,01 | 7/0,026 | - | 5 - 10 | 152/88[b)] | |
| 2 | tert.-Butylchlorid 600/6,48 | 20/0,075 | Tetrachlorethylen 162 | 2 - 3 | 667/85,3 | 96,5 |
| 3 | tert.-Butylchlorid 600/6,48 | 30/0,11 | Dichlormethan/ Acetylchlorid 133/0,8 | 2 - 3 | 682/97,3 | 96,6 |

a) bezogen auf Ausgangsstoffe RCl

b) zusätzlich 8,8 g exo-Norbornansäurebromid ≙ 4,3 % Ausbeute[a)]

## Ansprüche

1. Verfahren zur Herstellung von Carbonsäurehalogeniden der Formel I

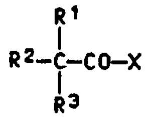

I,

in der $R^1$ Wasserstoff oder einen gegebenenfalls halogenierten Alkyl-oder Cycloalkylrest bedeutet, $R^2$ und $R^3$ gegebenenfalls halogenierte Alkyl- oder Cycloalkylreste darstellen, wobei $R^2$ und $R^3$ auch miteinander zu einem 5- bis 7-gliedrigen Ring verbunden sein können, und X für Halogen steht, durch Umsetzung von Alkylhalogeniden der Formel II

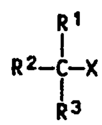

II

mit Kohlenmonoxid bei erhöhtem Druck in Gegenwart katalytischer Mengen einer Lewis-Säure, dadurch gekennzeichnet, daß man die Umsetzung in Gegenwart von

a) Aluminiumbromid als Katalysator oder

b) Aluminiumbromid oder -chlorid als Katalysator sowie in Gegenwart von Halogenkohlenwasserstoffen und Carbonsäurehalogeniden der Formel III

$$R^4–CH_2–CO–Hal \quad III$$

in der $R^4$ Wasserstoff oder einen $C_1$-$C_4$-Alkylrest bedeutet und Hal für Halogen steht,
vornimmt und gegebenenfalls den Katalysator anschließend durch Zugabe eines Carbonsäureamids zerstört.

2. Verfahren nach Anspruch 1, dadurch gekennzeichnet, daß man das Aluminiumbromid bzw. -chlorid in einer Menge von 0,005 bis 0,05 mol pro Mol Alkylhalogenid II verwendet.

3. Verfahren nach den Ansprüchen 1 und 2, dadurch gekennzeichnet, daß man als Lösungsmittel chlorierte Kohlenwasserstoffe verwendet.

4. Verfahren nach den Ansprüchen 1 bis 8, dadurch gekennzeichnet, daß man als Lösungsmittel chlorierte Alkane verwendet.

5. Verfahren nach den Ansprüchen 1 bis 4, dadurch gekennzeichnet, daß man 0,1 bis 2 mol Alkancarbonsäurehalogenid Je Mol Aluminiumhalogenid verwendet.

6. Verfahren nach din Ansprüchen 1 bis 5, dadurch gekennzeichnet, daß man als Alkancarbonsäurehalogenid Acetyl- oder Propionylchlorid verwendet.

7. Verfahren nach den Ansprüchen 1 bis 6, dadurch gekennzeichnet, daß man Alkylchloride oder -bromide umsetzt.

8. Verfahren nach den Ansprüchen 1 bis 7, dadurch gekennzeichnet, daß man die Umsetzung bei einem Druck von 10 bis 500 bar vornimmt.

9. Verfahren nach den Ansprüchen 1 bis 8, dadurch gekennzeichnet, daß man als Carbonsäureamid Dimethylformamid verwendet.

## Claims

1. A process for the preparation of a carbonyl halide of the formula I

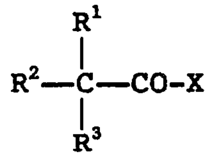 I,

where $R^1$ is hydrogen, alkyl, cycloalkyl, haloalkyl, or halocycloalkyl, $R^2$ and $R^3$ are each alkyl, cycloalkyl, haloalkyl or halocycloalkyl and may furthermore be bonded to one another to form a 5-membered to 7-membered ring, and X is halogen, by reacting an alkyl halide of the formula II

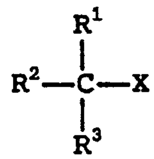 II

with carbon monoxide under superatmospheric pressure in the presence of a catalytic amount of a Lewis acid, wherein the reaction is carried out in the presence of

a) aluminum bromide as a catalyst or

b) aluminum bromide or chloride as a catalyst and in the presence of a halohydrocarbon and a carbonyl halide of the formula III

$$R^4\text{–}CH_2\text{–}CO\text{–}Hal \qquad III$$

where $R^4$ is hydrogen or $C_1$-$C_4$-alkyl and Hal is halogen
and the catalyst is then destroyed, if required, by adding a carboxamide.

2. A process as claimed in claim 1, wherein the aluminum bromide or chloride is used in an amount of from 0.005 to 0.05 mole per mole of alkyl halide II.

3. A process as claimed in either of claims 1 and 2, wherein a chlorohydrocarbon is used as the solvent.

4. A process as claimed in any of claims 1 to 3, wherein a chloroalkane is used as the solvent.

5. A process as claimed in any of claims 1 to 4, wherein from 0.1 to 2 moles of alkanecarbonyl halide are used per mole of aluminum halide.

6. A process as claimed in any of claims 1 to 5, wherein acetyl or propionyl chloride is used as the alkanecarbonyl halide.

7. A process as claimed in any of claims 1 to 6, wherein an alkyl chloride or bromide is reacted.

8. A process as claimed in any of claims 1 to 7, wherein the reaction is carried out under from 10 to 500 bar.

9. A process as claimed in any of claims 1 to 8, wherein dimethylformamide is used as the carboxamide.

**Revendications**

1. Procédé de préparation d'halogénures d'acides carboxyliques de formule I

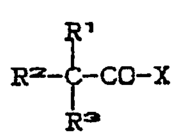 I,

dans laquelle $R^1$ représente un atome d'hydrogène ou un reste alkyle ou cycloalkyle éventuel élément halogéné, $R^2$ et $R^3$ représentent des restes alkyle ou cycloalkyle éventuellement halogénés, $R^2$ et $R^3$ pouvant également être liés l'un à l'autre pour former un noyau à 5-7 chaînons, et X est mis pour un atome d'halogène, par réaction d'halogénures d'alkyle de formule II

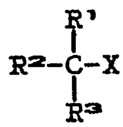

II

avec le monoxyde de carbone sous pression élevée en présence de quantités catalytiques d'un acide de Lewis, caractérisé en ce qu'on effectue la réaction en présence de

a) bromure d'aluminium comme catalyseur ou

b) bromure ou chlorure d'aluminium comme catalyseur ainsi qu'en présence d'hydrocarbures halogénés et d'halogénures d'acides carboxyliques de formule III

$$R^4\text{–}CH_2\text{–}CO\text{–}Hal \quad III$$

dans laquelle $R^4$ est un atome d'hydrogène ou un reste alkyle en $C_1$-$C_4$ et Hal est mis pour un atome d'halogène, et on détruit éventuellement ensuite le catalyseur par addition d'un carboxamide.

2. Procédé selon la revendication 1, caractérisé en ce qu'on utilise le bromure ou le chlorure d'aluminium à raison de 0,005 à 0,05 mole par mole d'halogénure d'alkyle II.

3. Procédé selon l'une ou l'autre des revendications 1 et 2, caractérisé en ce qu'on utilise comme solvant des hydrocarbures chlorés.

4. Procédé selon l'une quelconque des revendications 1 à 3, caractérisé en ce qu'on utilise comne solvant des alcanes chlorés,

5. Procédé selon l'une quelconque des revendications 1 à 4, caractérisé en ce qu'on utilise 0, 1 à 2 moles d'halogénure d'acide alcanecarboxylique par mole d'halogénure d'aluminium.

6. Procédé selon l'une quelconque des revendications 1 à 5, caractérisé en ce qu'on utilise comme halogénure d'acides alcanecarboxyliques du chlorure d'acétyle ou de propionyle.

7. Procédé selon l'une quelconque des revendications 1 à 6, caractérisé en ce qu'on fait réagir des chlorures ou des bromures d'alkyle.

8. Procédé selon l'une quelconque des revendications 1 à 7, caractérisé en ce qu'on mène la réaction à une pression de 10 à 500 bar.

9. Procédé selon l'une quelconque des revendications 1 à 8, caractérisé en ce qu'on utilise comme carboxamide du diméthylformamide.